# EUROPEAN PATENT SPECIFICATION

(11) **EP 0 888 374 B1**
(45) Date of publication and mention of the grant of the patent: **19.05.2010**
(21) Application number: 97905737.9
(22) Date of filing: 30.01.1997
(51) Int. Cl.: C12N 15/11, A61K 31/712, C12Q 1/68, C07H 21/04, C07H 21/02, A61K 31/7125, A61K 31/713

(54) **HIGH AFFINITY NUCLEIC ACID LIGANDS OF THE COMPLEMENT SYSTEM PROTEIN C1q**
NUKLEINSÄURELIGANDEN MIT HOHER AFFINITÄT FÜR DAS C1q PROTEIN DES KOMPLEMENTSYSTEMS
ACIDES NUCLEIQUES LIGANDS A FORTE AFFINITE POUR LA PROTEINE C1q DU SYSTEME COMPLEMENTAIRE

(30) Priority: 01.02.1996 US 595335
(43) Date of publication of application: 07.01.1999
(73) Proprietor: GILEAD SCIENCES, INC., Foster City, California 94404 (US)
(72) Inventor: BIESECKER, Gregory, Boulder, CO 80301 (US); GOLD, Larry, Boulder, CO 80302 (US)
(74) Representative: Clegg, Richard Ian
(86) International application number: PCT/US1997/001739
(87) International publication number: WO 1997/028178

(56) References cited:
- WO-A-92/14843
- WO-A-95/07364
- WO-A-97/42317
- US-A- 5 270 163
- ACTON S ET AL: "THE COLLAGENOUS DOMAINS OF MARCROPHAGE SCAVENGER RECEPTORS AND COMPLEMENT COMPONENT C1Q MEDIATE THEIR SIMILAR, BUT NOT IDENTICAL, BINDING SPECIFICITIES FOR POLYANIONIC LIGANDS" JOURNAL OF BIOLOGICAL CHEMISTRY, vol. 268, no. 5, 15 February 1993 (1993-02-15), pages 3530-3537, XP000770735 ISSN: 0021-9258
- PAUL MORGAN B: "PHYSIOLOGY AND PATHOPHYSIOLOGY OF COMPLEMENT: PROGRESS AND TRENDS" CRITICAL REVIEWS IN CLINICAL LABORATORY SCIENCES, vol. 32, no. 3, 1995, pages 265-298, XP002941854 ISSN: 1040-8363
- JIANG H ET AL: "DNA binds and activates Complement via residues 14-26 of the human C1q A chain" JOURNAL OF BIOLOGICAL CHEMISTRY, vol. 267, no. 35, 15 December 1992 (1992-12-15), pages 25597-25601, XP002240389 ISSN: 0021-9258

## Description

### FIELD OF THE INVENTION

Described herein are methods for identifying and preparing high-affinity Nucleic Acid Ligands to Complement System Proteins. The method utilized herein for identifying such Nucleic Acid Ligands is called SELEX, an acronym for Systematic Evolution of Ligands by EXponential enrichment. Described herein are methods for identifying and preparing high-affinity Nucleic Acid Ligands to the Complement System Protein C1q. This invention includes high affinity Nucleic Acid Ligands of C1q. Also disclosed are RNA ligands of C1q. Also disclosed are Nucleic Acid Ligands that inhibit and/or activate the Complement System. The oligonucleotides of the present invention are useful as pharmaceuticals or diagnostic agents.

### BACKGROUND OF THE INVENTION

The complement system comprises a set of at least 20 plasma and membrane proteins that act together in a regulated cascade system to attack extracellular forms of pathogens (47 and 48). There are two distinct enzymatic activation cascades, the classical and alternative pathways, and a nonenzymatic pathway known as the membrane attack pathway. The classical pathway is usually triggered by antibody bound to a foreign particle. It comprises several components, C1, C4, C2, C3, and C5 (listed by order in the pathway).

Initiation of the classical pathway of the Complement System occurs following binding and activation of the first complement component (C1) by both immune and non-immune activators (1). C1 comprises a calcium-dependent complex of components C1q, C1r and C1s, and is activated through binding of the C1q component. C1q contains six identical subunits and each subunit comprises three chains (the A, B and C chains). Each chain has a globular head region which is connected to a collagen-like tail. Binding and activation of C1q by antigen-antibody complexes occurs through the C1q head group region. Numerous non-antibody C1q activators, including proteins, lipids and nucleic acids (2), bind and activate through a distinct site on the collagen-like stalk region.

Non-antibody Clq protein activators include C-reactive protein (CRP) (3) and serum amyloid protein (SAP) (4); these will activate Clq when aggregated by binding to phospholipid or carbohydrate, respectively. Monomeric CRP or SAP do not activate C1q. C1q is also activated through binding to aggregated β-amyloid peptide (5,6), a component of plaques seen in Alzheimer's disease. C1q activation might also exacerbate the tissue damage associated with Alzheimer's disease. Other proteins which bind the C1q collagen-like region include collagen (49), fibronectin (12), laminin (13), fibrinogen and fibrin (14), HIV rsgp41 (15), actin (16) and tobacco glycoprotein (17).

C1q also binds and can be activated by anionic carbohydrates (18) including mucopolysaccharides (19), fucans (20), proteoglycans (21), and by lipids including lipopolysaccharide (LPS) (22,23). Both DNA (24-26) and RNA (27) can also bind and potentially activate C1q. Intracellular components which activate C1q include cellular and subcellular membranes (28-32), intermediate filaments (33), and actin (16). All of these interactions would recruit the classical pathway for protection against bacterial (or viral) infection, or as a response to tissue injury (34) in the absence of antibody.

A binding site for non-antibody activators including CRP (3), SAP (35), β-amyloid peptide (36), and DNA (37) has been localized to the amino terminous of Clq A chain at residues14-26. A synthetic peptide comprising this sequence effectively inhibits both binding and activation. The peptide 14-26 contains several basic residues and matches one of the heparin binding motifs (38,39). The peptide is also highly homologous with peptide 145-156 in collagen-tailed acetycholinesterase; this site is associated with heparin-sulfate basement membrane binding (40). A second Clq A chain site at residues 76-92 also might be involved in weaker binding; this site is at the junction of the globular head region and the collagen-like tail.

The second enzymatically activated cascade, known as the alternative pathway, is a rapid, antibody-independent route for the Complement System activation and amplification. The alternative pathway comprises several components, C3, Factor B, and Factor D. The nonenzymatic pathway, known as the membrane attack pathway, comprises components C6, C7, C8, and C9. The membrane attack pathway can directly lyse cells. In addition, it can stimulate cells such as endothelial cells and platelets without causing lysis.

The Complement System has an important role in defense against bacterial and viral infection, and possibly in immune surveillance against tumors. This is demonstrated most clearly in humans who are deficient in complement components. Individuals deficient in early components (C1, C4, C2 or C3) suffer from recurrent infections, while individuals deficient in late components (C5 through C9) are susceptible to *nisseria* infection. Complement classical pathway is activated on bacteria by antibody, or by binding of CRP or SAP, or by direct activation through LPS. Complement alternative pathway is activated through binding of C3 to the cell coat. Complement can be activated by viruses through antibody, and can also be activated on viral infected cells because these are recognized as foreign. In a similar way, transformed cells can be recognized as foreign and can be lysed by complement or targeted for immune clearance.

Activation of complement can and has been used for therapeutic purposes. Antibodies which were produced against tumor cells were then used to activate complement and cause tumor rejection. Also, complement is used together with polyclonal or monoclonal antibody to eliminate unwanted lymphocytes. For example, anti-lymphocyte globulin or monoclonal anti-T-cell antibody are used prior to organ transplantation to eliminate lymphocytes which would otherwise mediate rejection.

Although the Complement System has an important role in the maintenance of health, it has the potential to cause or contribute to disease. The complement system has been implicated in numerous renal, rheumatological, neurological, dermatological, hematological, vascular/pulmonary, allergy, infectious, biocompatibility/shock and other diseases or conditions (see 48 and 50). The Complement System is not necessarily the only cause of the disease state, but it may be one of several factors, each of which contributes to pathogenesis.

Several pharmaceuticals have been developed that inhibit the Complement System in vivo; however, many cause toxicity or are poor inhibitors (48). Heparins, K76COOH, and nafamstat mesilate have been shown to be effective in animal studies (48). Recombinant forms of naturally occurring inhibitors of the Complement System have been developed or are under consideration, and these include the membrane regulatory proteins Complement Receptor 1 (CR1), Decay Accelerating Factor (DAF), Membrane Cofactor Protein (MCP), and CD59.

C5 is an attractive target for the development of a Complement System inhibitor, as both the classical and alternative pathways converge at component C5 (50). Matis and Rollins (1995) have developed C5-specific monoclonal antibodies as an antiinflammatory biopharmaceutical.

C3 is an attractive target for the development of a Complement System inhibitor, as it is common to both pathways. Controlling C3 limits most biological activities of the Complement System. Most natural inhibitors, including DAF, MCP, CR1 and Factor H, target C3.

### SELEX

A method for the *in vitro* evolution of Nucleic Acid molecules with highly specific binding to Target molecules has been developed. This method, Systematic Evolution of Ligands by EXponential enrichment, termed SELEX, is described in United States Patent Application Serial No. 07/536,428, entitled "Systematic Evolution of Ligands by Exponential Enrichment," now abandoned, United States Patent Application Serial No. 07/714,131, filed June 10, 1991, entitled "Nucleic Acid Ligands," now United States Patent No. 5,475,096, United States Patent Application Serial No. 07/931.473, filed August 17, 1992, entitled "Nucleic Acid Ligands," now United States Patent No. 5,270,163 (see also PCT/US91/04078). Each of these applications, collectively referred to herein as the SELEX Patent Applications, describes a fundamentally novel method for making a Nucleic Acid Ligand to any desired Target molecule.

The SELEX method involves selection from a mixture of candidate oligonucleotides and step-wise iterations of binding, partitioning and amplification, using the same general selection scheme, to achieve virtually any desired criterion of binding affinity and selectivity. Starting from a mixture of Nucleic Acids, preferably comprising a segment of randomized sequence, the SELEX method includes steps of contacting the mixture with the Target under conditions favorable for binding, partitioning unbound Nucleic Acids from those Nucleic Acids which have bound specifically to Target molecules, dissociating the Nucleic Acid-Target complexes, amplifying the Nucleic Acids dissociated from the Nucleic Acid-Target complexes to yield a ligand-enriched mixture of Nucleic Acids, then reiterating the steps of binding, partitioning, dissociating and amplifying through as many cycles as desired to yield highly specific, high affinity Nucleic Acid Ligands to the Target molecule.

The basic SELEX method has been modified to achieve a number of specific objectives. For example, United States Patent Application Serial No. 07/960,093, filed October 14, 1992, entitled "Method for Selecting Nucleic Acids on the Basis of Structure," describes the use of SELEX in conjunction with gel electrophoresis to select Nucleic Acid molecules with specific structural characteristics, such as bent DNA. United States Patent Application Serial No. 08/123,935, filed September 17, 1993, entitled "Photoselection of Nucleic Acid Ligands" describes a SELEX based method for selecting Nucleic Acid Ligands containing photoreactive groups capable of binding and/or photocrosslinking to and/or photoinactivating a Target molecule. United States Patent Application Serial No. 08/134,028, filed October 7, 1993, entitled "High-Affinity Nucleic Acid Ligands That Discriminate Between Theophylline and Caffeine," describes a method for identifying highly specific Nucleic Acid Ligands able to discriminate between closely related molecules, termed Counter-SELEX. United States Patent Application Serial No. 08/143,564, filed October 25, 1993, entitled "Systematic Evolution of Ligands by EXponential Enrichment: Solution SELEX," describes a SELEX-based method which achieves highly efficient partitioning between oligonucleotides having high and low affinity for a Target molecule. United States Patent Application Serial No. 07/964,624, filed October 21, 1992, entitled "Methods of Producing Nucleic Acid Ligands," now United States Patent No. 5,496,938, describes methods for obtaining improved Nucleic Acid Ligands after SELEX has been performed. United States Patent Application Serial No. 08/400,440, filed March 8, 1995, entitled "Systematic Evolution of Ligands by EXponential Enrichment: Chemi-SELEX," describes methods for covalently linking a ligand to its Target.

The SELEX method encompasses the identification of high-affinity Nucleic Acid Ligands containing modified nucleotides conferring improved characteristics on the ligand, such as improved *in vivo* stability or improved delivery characteristics. Examples of such modifications include chemical substitutions at the ribose and/or phosphate and/or base positions. SELEX-identified Nucleic Acid Ligands containing modified nucleotides are described in United States Patent Application Serial No. 08/117,991, filed September 8, 1993, entitled "High Affinity Nucleic Acid Ligands Containing Modified Nucleotides," that describes oligonucleotides containing nucleotide derivatives chemically modified at the 5- and 2'-positions of pyrimidines. United States Patent Application Serial No. 08/134,028, *supra,* describes highly specific Nucleic Acid Ligands containing one or more nucleotides modified with 2'-amino (2'-NH₂), 2'-fluoro (2'-F), and/or 2'-O-methyl (2'-OMe). United States Patent Application Serial No. 08/264,029, filed June 22, 1994, entitled "Novel Method of Preparation of 2' Modified Pyrimidine Intramolecular Nucleophilic Displacement," describes oligonucleotides containing various 2'-modified pyrimidines.

The SELEX method encompasses combining selected oligonucleotides with other selected oligonucleotides and non-oligonucleotide functional units as described in United States Patent Application Serial No. 08/284,063, filed August 2, 1994, entitled "Systematic Evolution of Ligands by Exponential Enrichment: Chimeric SELEX" and United States Patent Application Serial No. 08/234,997, filed April 28, 1994, entitled "Systematic Evolution of Ligands by Exponential Enrichment: Blended SELEX," respectively. These applications allow the combination of the broad array of shapes and other properties, and the efficient amplification and replication properties, of oligonucleotides with the desirable properties of other molecules. Each of the above described patent applications which describe modifications of the basic SELEX procedure are specifically incorporated by reference herein in their entirety.

Acton et al (The Journal of Biological Chemistry Vol. 268, No.5, Feb 25, pp 3530-3537, 1993) describes binding between complement component C1q and a variety of polyanions, such as polyribonucleotides poly(I) and poly(G).

Morgan (Critical Reviews in Clinical Laboratory Services 32(3);265-298 (1995)) discusses the use of complement inhibitory molecules in therapy of complement-mediated disease.

WO 92/14843 A describes a method for generating nucleic acid ligands and discusses their use in diagnostics, therapeutics and research.

### BRIEF SUMMARY OF THE INVENTION

The present disclosure includes methods of identifying and producing Nucleic Acid Ligands to Complement System Proteins and homologous proteins and the Nucleic Acid Ligands so identified and produced. By substantially homologous it is meant a degree of amino acid sequence identity of 80% or more. Exemplified herein is a method of identifying and producing Nucleic Acid Ligands to C1q and the Nucleic Acid Ligands so produced. Nucleic acid ligand sequences are provided that are capable of binding specifically to C1q. In particular, RNA sequences are provided that are capable of binding specifically the C1q. Specifically disclosed are the RNA ligand sequences shown in Table 2 (SEQ ID NOS:5-20). Also included in the invention are Nucleic Acid Ligands that inhibit the function of proteins of the complement system. Included in the invention herein are RNA ligands that inhibit the function of Clq. Also included are Nucleic Acid Ligands that inhibit and/or activate the Complement System.

Further disclosed is a method of identifying Nucleic Acid Ligands and Nucleic Acid Ligand sequences to Complement System Proteins comprising the steps of (a) preparing a Candidate Mixture of Nucleic Acids, (b) contacting the Candidate Mixture of Nucleic Acids with a Complement System Protein, (c) partitioning between members of said Candidate Mixture on the basis of affinity to said Complement System Protein, and (d) amplifying the selected molecules to yield a mixture of Nucleic Acids enriched for Nucleic Acid sequences with a relatively higher affinity for binding to said Complement System Protein.

Also disclosed is a method of identifying Nucleic Acid Ligands and Nucleic Acid Ligand sequences to C1q comprising the steps of (a) preparing a Candidate Mixture of Nucleic Acids, (b) contacting the Candidate Mixture of Nucleic Acids with C1q, (c) partitioning between members of said Candidate Mixture on the basis of affinity to C1q, and (d) amplifying the selected molecules to yield a mixture of Nucleic Acids enriched for Nucleic Acid sequences with a relatively higher affinity for binding to C1q.

More specifically, the present disclosure includes the RNA ligands to C1q, identified according to the above-described method, including those ligands shown in Table 2 (SEQ ID NOS:5-20). Also disclosed are RNA ligands to C1q that are substantially homologous to any of the given ligands and that have substantially the same ability to bind C1q and inhibit the function of C1q. Further disclosed are Nucleic Acid Ligands to C1q that have substantially the same structural form as the ligands presented herein and that have substantially the same ability to bind said C1q and inhibit the function of said C1q.

The present disclosure also includes modified nucleotide sequences based on the RNA ligands identified herein and mixtures of the same.

### DETAILED DESCRIPTION OF THE INVENTION

### DEFINITIONS:

"Nucleic Acid Ligand" as used herein is a non-naturally occurring Nucleic Acid having a desirable action on a Target. A desirable action includes, but is not limited to, binding of the Target, catalytically changing the Target, reacting with the Target in a way which modifies/alters the Target or the functional activity of the Target, covalently attaching to the Target as in a suicide inhibitor, facilitating the reaction between the Target and another molecule. In the preferred embodiment, the action has specific binding affinity for a Target molecule, such Target molecule being a three dimensional chemical structure other than a polynucleotide that binds to the Nucleic Acid Ligand through a mechanism which predominantly depends on Watson/Crick base pairing or triple helix binding, wherein the Nucleic Acid Ligand is not a Nucleic Acid having the known physiological function of being bound by the Target molecule. Nucleic Acid Ligands include Nucleic Acids that are identified from a Candidate Mixture of Nucleic Acids, said Nucleic Acid Ligand being a ligand of a given Target by the method comprising: a) contacting the Candidate Mixture with the Target, wherein Nucleic Acids having an increased affinity to the Target relative to the Candidate Mixture may be partitioned from the remainder of the Candidate Mixture; b) partitioning the increased affinity Nucleic Acids from the remainder of the Candidate Mixture; and c) amplifying the increased affinity Nucleic Acids to yield a ligand-enriched mixture of Nucleic Acids.

"Candidate Mixture" is a mixture of Nucleic Acids of differing sequence from which to select a desired ligand. The source of a Candidate Mixture can be from naturally-occurring Nucleic Acids or fragments thereof, chemically synthesized Nucleic Acids, enzymatically synthesized Nucleic Acids or Nucleic Acids made by a combination of the foregoing techniques. In a preferred embodiment, each Nucleic Acid has fixed sequences surrounding a randomized region to facilitate the amplification process.

"Nucleic Acid" means either DNA, RNA, single-stranded or double-stranded and any chemical modifications thereof. Modifications include, but are not limited to, those which provide other chemical groups that incorporate additional charge, polarizability, hydrogen bonding, electrostatic interaction, and fluxionality to the Nucleic Acid Ligand bases or to the Nucleic Acid Ligand as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases isocytidine and isoguanidine and the like. Modifications can also include 3' and 5' modifications such as capping.

"SELEX" methodology involves the combination of selection of Nucleic Acid Ligands which interact with a Target in a desirable manner, for example binding to a protein, with amplification of those selected Nucleic Acids. Iterative cycling of the selection/amplification steps allows selection of one or a small number of Nucleic Acids which interact most strongly with the Target from a pool which contains a very large number of Nucleic Acids. Cycling of the selection/amplification procedure is continued until a selected goal is achieved. In the present invention, the SELEX methodology is employed to obtain Nucleic Acid Ligands to C1q.

The SELEX methodology is described in the SELEX Patent Applications.

"Target" means any compound or molecule of interest for which a ligand is desired. A Target can be a protein, peptide, carbohydrate, polysaccharide, glycoprotein, hormone, receptor, antigen, antibody, virus, substrate, metabolite, transition state analog, cofactor, inhibitor, drug, dye, nutrient, growth factor, etc. without limitation. In this application, the Target is a Complement System Protein, preferably C1q.

"Complement System Protein" means any protein or component of the Complement System including, but not limited to, C1, C1q, C1r, C1s, C2, C3, C3a, C3b, C4, C4a, C5, C5a, C5b, C6, C7, C8, C9, Factor B (B), Factor D (D), Factor H (H), and receptors thereof, and other soluble and membrane inhibitors/control proteins.

"Complement System" is a set of plasma and membrane proteins that act together in a regulated cascade system to attack extracellular forms of pathogens or infected or transformed cells, and in clearance of immune reactants or cellular debris. The Complement System can be activated spontaneously on certain pathogens or by antibody binding to the pathogen. The pathogen becomes coated with Complement System Proteins (opsonized) for uptake and destruction. The pathogen can also be directly lysed and killed. Similar mechanisms target infected, transformed, or damaged cells. Complement also participates in clearance of immune and cellular debris.

SELEX is described in U.S. Patent Application Serial No. 07/536,428, entitled Systematic Evolution of Ligands by EXponential Enrichment, now abandoned, U.S. Patent Application Serial No. 07/714,131, filed June 10, 1991, entitled Nucleic Acid Ligands, now United States Patent No. 5,475,096, United States Patent Application Serial No. 07/931,473, filed August 17, 1992, entitled Nucleic Acid Ligands, now United States Patent No. 5,270,163, (see also PCT/US91/04078). These applications, are collectively called the SELEX Patent Applications.

In its most basic form, the SELEX process may be defined by the following series of steps:
1) A Candidate Mixture of Nucleic Acids of differing sequence is prepared. The Candidate Mixture generally includes regions of fixed sequences (i.e., each of the members of the Candidate Mixture contains the same sequences in the same location) and regions of randomized sequences. The fixed sequence regions are selected either: (a) to assist in the amplification steps described below, (b) to mimic a sequence known to bind to the Target, or (c) to enhance the concentration of a given structural arrangement of the Nucleic Acids in the Candidate Mixture. The randomized sequences can be totally randomized (i.e., the probability of finding a base at any position being one in four) or only partially randomized (e.g., the probability of finding a base at any location can be selected at any level between 0 and 100 percent).
2) The Candidate Mixture is contacted with the selected Target under conditions favorable for binding between the Target and members of the Candidate Mixture. Under these circumstances, the interaction between the Target and the Nucleic Acids of the Candidate Mixture can be considered as forming Nucleic Acid-Target pairs between the Target and those Nucleic Acids having the strongest affinity for the Target.
3) The Nucleic Acids with the highest affinity for the Target are partitioned from those Nucleic Acids with lesser affinity to the Target. Because only an extremely small number of sequences (and possibly only one molecule of Nucleic Acid) corresponding to the highest affinity Nucleic Acids exist in the Candidate Mixture, it is generally desirable to set the partitioning criteria so that a significant amount of the Nucleic Acids in the Candidate Mixture (approximately 5-50%) are retained during partitioning.
4) Those Nucleic Acids selected during partitioning as having the relatively higher affinity to the Target are then amplified to create a new Candidate Mixture that is enriched in Nucleic Acids having a relatively higher affinity for the Target.
5) By repeating the partitioning and amplifying steps above, the newly formed Candidate Mixture contains fewer and fewer weakly binding sequences, and the average degree of affinity of the Nucleic Acids to the Target will generally increase. Taken to its extreme, the SELEX process will yield a Candidate Mixture containing one or a small number of unique Nucleic Acids representing those Nucleic Acids from the original Candidate Mixture having the highest affinity to the Target molecule.

The SELEX Patent Applications describe and elaborate on this process in great detail. Included are Targets that can be used in the process; methods for partitioning Nucleic Acids within a Candidate Mixture; and methods for amplifying partitioned Nucleic Acids to generate enriched Candidate Mixture. The SELEX Patent Applications also describe ligands obtained to a number of target species, including both protein Targets where the protein is and is not a Nucleic Acid binding protein.

The Nucleic Acid Ligands to a Complement System Protein can be complexed with a lipophilic compound (e.g., cholesterol) or attached to or encapsulated in a complex comprised of lipophilic components (e.g., a liposome). U.S. Patent Application No. 08/434,465, filed May 4, 1995, entitled "Nucleic Acid Ligand Complexes," describes a method for preparing a therapeutic or diagnostic complex comprised of a Nucleic Acid Ligand and a lipophilic compound or a non-immunogenic, high molecular weight compound.

The methods described herein and the Nucleic Acid Ligands identified by such methods are useful for both therapeutic and diagnostic purposes. Therapeutic uses include the treatment or prevention of diseases or medical conditions in human patients, specifically diseases or conditions caused by activation of the complement system. The Complement System does not have to be the only cause of the disease state, but it may be one of several factors, each of which contributes to pathogenesis. Such diseases or conditions include, but are not limited to, renal diseases, such as lupus nephritis, membranoproliferative glomerulonephritis (MPGN), membranous nephritis, IgA nephropathy; rheumatological diseases, such as rheumatoid arthritis, systemic lupus erythematosus (SLE), Behcet's syndrome, juvenile rheumatoid, Sjögren's syndrome, systemic sclerosis; neurological diseases, such as myasthenia gravis, multiple sclerosis, cerebral lupus, Guillain-Barré syndrome, Alzheimer's disease; dermatological diseases, such as Pemphigus/pemphigoid, phototoxic reactions, vasculitis, thermal burns; hematological diseases, such as paroxysmal nocturnal hemoglobinuria (PNH), hereditary erythroblastic multinuclearity with positive acidified serum lysis test (HEMPAS), idiopathic thrombocytopenic purpura (ITP); biocompatibility/shock diseases, such as post-bypass syndrome, adult respiratory distress syndrome (ARDS), catheter reactions, anaphylaxis, transplant rejection, pre-eclampsia, hemodialysis, platelet storage; vascular/pulmonary diseases, such as atherosclerosis, myocardial infarction, stroke, reperfusion injury; allergy, such as anaphylaxis, asthma, skin reactions; infection, such as septic shock, viral infection, bacterial infection; and other conditions, such as atheroma, bowel inflammation, thyroiditis, and infertility, paroxysmal nocturnal hemoglobinuria (PNH), hemolytic anemia.

In other instances, the activation of the Complement System is desirable in the treatment or prevention of diseases or medical conditions in human patients. For example, the activation of the Complement System is desirable in treating bacterial or viral infections and malignancies. In addition, the activation of the Complement System on T cells prior to transplantation could prevent rejection of an organ or tissue by eliminating the T cells that mediate the rejection.

Furthermore, Nucleic Acid Ligands that bind to cell surface Targets could be made more efficient by giving them the ability to activate the Complement System. Nucleic Acid binding would then both inhibit a Target function, and also eliminate the cell, for example, by the membrane attack complex lysis and cell clearance through opsonization. Nucleic Acid Ligands could activate the Complement System through either the classical or the alternative pathways. C1q Nucleic Acid Ligands can be conjugated to other structures that target a cell surface component. For example, C1q Nucleic Acid Ligands can be conjugated to antibodies to cell targets, cytokines, growth factors, or a ligand to a cell receptor. This would allow the C1q Nucleic Acid Ligands to multimerize on the targeted cell surface and activate the Complement System, thereby killing the cell.

The prototype classical pathway activators are immune aggregates, which activate the Complement System through binding to globular head groups on the C1q component. Generally, binding of two or more Fc domains to C1q is required; pentameric IgM is an especially efficient activator. In contrast, nucleic acids can activate through binding at a separate site on the C1q collagen-like tail region. This site also binds to a variety of other non-antibody activators including C-reactive protein, serum amyloid protein, endotoxin, β-amyloid peptide 1-40, and mitochondrial membranes. As with immunoglobulin, these non-antibody activators need to be multimerized to activate.

Nucleic Acid Ligands that bind to sites on the collagen-like region of C1q may also become activators when aggregated. Such a complement-activating aggregate may be lytic if formed on a cell surface, such as binding to a tumor-specific antigen (TSA) or to a leukocyte antigen. The extent of Nucleic Acid Ligand-mediated activation increases with the extent of Nucleic Acid Ligand aggregation (i.e., multiplicity of Nucleic Acid Ligand-C1q interaction). The Complement System-mediated killing is especially specific if the Nucleic Acid Ligands circulate as monomers which do not activate, but become activators when they are multimerized on the targeted cell surface. As with any Complement System activation, the extent and specificity is determined by the amount of C3 deposited onto the targeted cell. Deposited C3 forms an enzyme convertase that cleaves C5 and initiates membrane attack complex formation; C3 is also the classical serum opsonin for targeting phagocytic ingestion.

The prototype alternative pathway activators are repeating carbohydrate units including bacterial and yeast cell walls, fucoidin, and Sepharose, or glycolipids such as endotoxin or the glycocalyx. Nucleic Acid Ligands could activate the alternative pathway by aggregating the C3 component on the cell surface. Depositing C3 on a cell promotes Factor B binding and alternative pathway C3 convertase formation. Binding of a Nucleic Acid Ligand to C3 blocks binding of the inhibitor Factor H, and prevents C3b decay. This would also increase C3 convertase formation and alternative path activation. C3 Nucleic Acid Ligands may have this activity since heparin binds activated C3 and can promote alternative pathway activation. Binding of Nucleic Acid Ligands to C3 blocks binding to C3 of the membrane-associated inhibitors CR1, CR2, MCP, DAF, preventing C3b convertase decay and stimulating alternative pathway activation. This alternative pathway mechanism can be as efficient as C1q-dependent activation in cell killing and lysis.

Nucleic Acid Ligand-mediated Complement System cell killing could be employed in several ways, for example, by: a) direct killing of tumor cells; b) lysis of targeted microorganisms or infected cells; c) elimination of lymphocytes or lymphocyte subsets. Nucleic Acid Ligands could replace antibodies currently used for these purposes.

Diagnostic utilization may include both in vivo or in vitro diagnostic applications. The SELEX method generally, and the specific adaptations of the SELEX method taught and claimed herein specifically, are particularly suited for diagnostic applications. The SELEX method identifies Nucleic Acid Ligands that are able to bind targets with high affinity and with surprising specificity. These characteristics are, of course, the desired properties one skilled in the art would seek in a diagnostic ligand.

The Nucleic Acid Ligands of the present invention may be routinely adapted for diagnostic purposes according to any number of techniques employed by those skilled in the art. Diagnostic agents need only be able to allow the user to identify the presence of a given target at a particular locale or concentration. Simply the ability to form binding pairs with the target may be sufficient to trigger a positive signal for diagnostic purposes. Those skilled in the art would also be able to adapt any Nucleic Acid Ligand by procedures known in the art to incorporate a labeling tag in order to track the presence of such ligand. Such a tag could be used in a number of diagnostic procedures. The Nucleic Acid Ligands to C1q described herein may specifically be used for identification of the C1q protein.

The SELEX process provides high affinity ligands of a target molecule. This represents a singular achievement that is unprecedented in the field of Nucleic Acids research. The present invention applies the SELEX procedure to the specific target of C1q, which is part of the first component (C1) of the classical pathway of complement activation. In the Example section below, the experimental parameters used to isolate and identify the Nucleic Acid Ligands to C1q are described.

In order to produce Nucleic Acids desirable for use as a pharmaceutical, it is preferred that the Nucleic Acid Ligand (1) binds to the target in a manner capable of achieving the desired effect on the target; (2) be as small as possible to obtain the desired effect; (3) be as stable as possible; and (4) be a specific ligand to the chosen target. In most situations, it is preferred that the Nucleic Acid Ligand have the highest possible affinity to the target.

Pharmaceutical agents, which include, but are not limited to, small molecules, antisense oligonucleotides, nucleosides, and polypeptides can activate the Complement System in an undesirable manner. Nucleic Acid Ligands to Complement System Proteins could be used as a prophylactic by transiently inhibiting the Complement System, so that a pharmaceutical agent could be administered and achieve a therapeutically effective amount without eliciting the undesirable side effect of activating the Complement System.

In co-pending and commonly assigned U.S. Patent Application Serial No. 07/964,624, filed October 21, 1992 ('624), now United States Patent No. 5,496,938, methods are described for obtaining improved Nucleic Acid Ligands after SELEX has been performed.

In the present invention, a SELEX experiment was performed in order to identify RNA with specific high affinity for C1q from a degenerate library containing 50 random positions (50N) (Example 1). This invention includes the specific RNA ligands to C1q shown in Table 2 (SEQ ID NOS:5-20), identified by the method described in Example 1. This invention further includes RNA ligands to C1q which inhibit the function of C1q. The scope of the ligands covered by this invention extends to all Nucleic Acid Ligands of C1q, modified and unmodified, identified according to the SELEX procedure. More specifically, this invention includes Nucleic Acid sequences that are substantially homologous to the ligands shown in Table 2 (SEQ ID NOS:5-20). By substantially homologous it is meant a degree of primary sequence homology in excess of 70%, most preferably in excess of 80%. A review of the sequence homologies of the ligands of C1q shown in Table 2 (SEQ ID NOS:5-20) shows that sequences with little or no primary homology may have substantially the same ability to bind C1q. For these reasons, this invention also includes Nucleic Acid Ligands that have substantially the same structure and ability to bind C1q as the Nucleic Acid Ligands shown in Table 2 (SEQ ID NOS:5-20). Substantially the same ability to bind C1q means that the affinity is within one or two orders of magnitude of the affinity of the ligands described herein. It is well within the skill of those of ordinary skill in the art to determine whether a given sequencesubstantially homologous to those specifically described herein -- has substantially the same ability to bind C1q.

One potential problem encountered in the therapeutic, prophylactic, and in vivo diagnostic use of Nucleic Acids is that oligonucleotides in their phosphodiester form may be quickly degraded in body fluids by intracellular and extracellular enzymes such as endonucleases and exonucleases before the desired effect is manifest. Certain chemical modifications of the Nucleic Acid Ligand can be made to increase the in vivo stability of the Nucleic Acid Ligand or to enhance or to mediate the delivery of the Nucleic Acid Ligand. See, e.g., U.S. Patent Application Serial No. 08/117,991, filed September 9, 1993, entitled High Affinity Nucleic Acid Ligands Containing Modified Nucleotides and U.S. Patent Application Serial No. 08/434,465, filed May 4, 1995, entitled Nucleic Acid Ligand Complexes. Modifications of the Nucleic Acid Ligands contemplated in this invention include, but are not limited to, those which provide other chemical groups that incorporate additional charge, polarizability, hydrophobicity, hydrogen bonding, electrostatic interaction, and fluxionality to the Nucleic Acid Ligand bases or to the Nucleic Acid Ligand as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or alkyl phosphate modifications, methylations, unusual base-pairing combinations such as the isobases isocytidine and isoguanidine and the like. Modifications can also include 3' and 5' modifications such as capping.

The modifications can be pre- or post- SELEX modifications. Pre-SELEX modifications yield Nucleic Acid Ligands with both specificity for their SELEX Target and improved in vivo stability. Post-SELEX modifications made to 2'-OH Nucleic Acid Ligands can result in improved in vivo stability without adversely affecting the binding capacity of the Nucleic Acid Ligand. Described herein are Nucleic Acid Ligands that were 2'-NH₂ modified and incorporated into the SELEX process.

Other modifications are known to one of ordinary skill in the art. Such modifications may be made post-SELEX (modification of previously identified unmodified ligands) or by incorporation into the SELEX process.

As described above, because of their ability to selectively bind C1q, the Nucleic Acid Ligands to C1q described herein are useful as pharmaceuticals. This invention, therefore, also includes a method for treating Complement System-mediated diseases by administration of a Nucleic Acid Ligand capable of binding to a Complement System Protein or homologous proteins. Certain diseases or conditions, such as Alzheimer's disease or myocardial infarction activate C1q through the collagen-like region. In Alzheimer disease, β-amyloid activates C1q. Structures in heart muscle that are exposed during myocardial infarction, such as intermediate filaments, mitochondial membranes or actin activate C1q. Thus, the Nucleic Acid Ligands of the present invention may be useful in treating Alzheimer's disease or myocardial infarction.

Therapeutic compositions of the Nucleic Acid Ligands may be administered parenterally by injection, although other effective administration forms, such as intraarticular injection, inhalant mists, orally active formulations, transdermal iontophoresis or suppositories, are also envisioned. One preferred carrier is physiological saline solution, but it is contemplated that other pharmaceutically acceptable carriers may also be used. In one preferred embodiment, it is envisioned that the carrier and the ligand constitute a physiologically-compatible, slow release formulation. The primary solvent in such a carrier may be either aqueous or non-aqueous in nature. In addition, the carrier may contain other pharmacologically-acceptable excipients for modifying or maintaining the pH, osmolarity, viscosity, clarity, color, sterility, stability, rate of dissolution, or odor of the formulation. Similarly, the carrier may contain still other pharmacologically-acceptable excipients for modifying or maintaining the stability, rate of dissolution, release, or absorption of the ligand. Such excipients are those substances usually and customarily employed to formulate dosages for parental administration in either unit dose or multi-dose form.

Once the therapeutic composition has been formulated, it may be stored in sterile vials as a solution, suspension, gel, emulsion, solid, or dehydrated or lyophilized powder. Such formulations may be stored either in a ready to use form or requiring reconstitution immediately prior to administration. The manner of administering formulations containing Nucleic Acid Ligands for systemic delivery may be via subcutaneous, intramuscular, intravenous, intranasal or vaginal or rectal suppository.

In the Examples that follow, the use of SELEX methodology to identify high affinity RNA ligands to C1q is described.

The following Examples are provided to explain and illustrate the present invention and are not intended to be limiting of the invention. Example 1 describes the various materials and experimental procedures used in Example 2. Example 2 describes the 2'-NH₂ RNA ligands to C1q. Example 3 describes the generation of Nucleic Acid Ligands of Complement System Protein C3. Example 4 describes the generation of Nucleic Acid Ligands of Complement System Protein C5. Example 5 describes the activation of the Complement System.

### EXAMPLE 1. EXPERIMENTAL PROCEDURES

This example provides general procedures followed and incorporated in Example 2 for the identification of 2'-NH₂ RNA ligands to C1q.

### A. Biochemical

C1q and C4-deficient guinea pig sera were obtained from Quidel (San Diego, CA). Bovine serum albumin (BSA), rabbit anti-BSA, CRP, SAP and β-amyloid peptides 1-40 and 1-42 were obtained from Sigma (St. Louis, MO). Nucleotides GTP, ATP, and deoxynucleotides were obtained from Pharmacia (Uppsala, Sweden). Taq polymerase was obtained from Perkin-Elmer (Norwalk, CT). Modified nucleotides 2NH₂-CTP and 2'NH₂-UTP, were prepared as described (42). Avian reverse transcriptase was obtained from Life Sciences (St. Petersburg, FL) and T7 RNA polymerase from USB (Cleveland, OH). Nitrocellulose filters were obtained from Millipore (Bedford, MA). All chemicals were the highest grade available.

### B. RNA SELEX procedures

The SELEX procedure has been described in detail in the SELEX Patent Applications (see also 42,43). Briefly, a DNA template was synthesized with a 5' fixed region containing the T7 promoter, followed by a 50n stretch of random sequence, and then with a 3'-fixed region (Table I; SEQ ID NOS: 1-2). For the initial round of SELEX, 1 nmole (~10¹⁴ unique sequences) of RNA was *in vitro* transcribed by T7 polymerase (44) using mixed GTP/ATP and 2'-NH₂-CTP/UTP nucleotides, and with the addition of α-[³²P]-ATP. For this and subsequent SELEX rounds, the RNA was purified by electrophoresis on 8% acrylamide gels with 7 M urea, 10 mM Tris-Borate, 2 mM EDTA, pH 8.3 running buffer. After autoradiography, the band containing labeled 2'-NH₂-RNA transcript was excised and frozen at -70°C, then 400 µl of 100 mM NaCl, 2 mM EDTA was added, the gel was mashed, and the slurry was spun through 2 cm of glass-wool (Rnase-free - Alltech Associates, Deerfield, IL) and two nitrocellulose filters. The RNA was precipitated by addition of 1/5 vol of 6.6 M NH₄OAc, pH 7.7, plus 2 vol of ethanol. The pellet was washed twice with 80% ethanol, and taken to dryness. The dry RNA pellet was dissolved in phosphate buffered saline (45) containing 1 mM MgCl₂ (MgPBS).

For each round of SELEX, the RNA was incubated with C1q in MgPBS for 10 min at 37°C. Then the sample was filtered through a 43 mm nitrocellulose filter, and the filter was washed with 10 ml of MgPBS. For some rounds, the diluted RNA was pre-soaked with nitrocellulose filters overnight to reduce background. Four samples were run in parallel for most rounds with lesser amounts (chosen to be in suitable range to measure binding) of both RNA and C1q to measure binding Kd for each sample. In addition, at each round, a sample of RNA was filtered without protein to determine background.

Filters were air-dried, sliced into strips, counted, and then extracted for 60 min at 37°C with 400 µl of 1% SDS, 0.5 mg/ml Proteinase K (Boehringer Mannheim, Indianapolis, IN), 1.5 mM DTT, 10 mM EDTA, 0.1 M Tris, pH 7.5, with addition of 40 µg tRNA carrier. The aqueous RNA was extracted with phenol, phenol/chloroform (1:1), chloroform and then precipitated following addition of NH₂OAc/EtOH as above. The RNA was reverse transcribed in a volume of 50 µl for between 1 hr and overnight. The DNA was PCR amplified with specific primers (Table 1; SEQ ID NOS:3-4) in a volume of 500 µl for 12-14 cycles, and then phenol/chloroform extracted and NaOAc/EtOH precipitated. The DNA pellet was taken up in H₂O, and an aliquot was T7 transcribed for the next round of SELEX.

### C. Cloning.

DNA from the 14th round was PCR amplified a second time and then purified by FPLC on a monoQ column (Pharmacia, Piscataway, NJ), with a gradient from 50 mM Tris, pH 8.0 to 1 M NaCl, 50 mM Tris, pH 8.0. The DNA was cloned into a pCR-Script SK (+) vector (Stratagene, La Jolla, CA), and colonies were picked for overnight growth and plasmid mini-preps (PERFECTprep, 5'-3', Boulder, CO). The purified plasmids were PCR amplified with 3' and 5' primers (as above), and products were analyzed by agarose gel electrophoresis (45). DNA was T7 transcribed with α-[³²P]-ATP to prepare radioactive RNA for binding analysis and without radiolabel to prepare RNA for inhibition studies.

### D. Sequencing

Plasmids purified using the PERFECTprep kit were sequenced with the Sequenase Verson 2.0 kit (USB, Cleveland, OH) with addition of α-[³⁵S]-ATP. The samples were run on 12% acrylamide gels in 7 M urea, TBE, then gels were dried and exposed to film for determining the sequence.

### E. Binding Assays

Individual cloned DNA was T7 transcribed with α-[³²P]-ATP and the full length [³²P]-2'-NH₂-RNA was gel-purified (as above). RNA was suspended at approximately 5,000 cpm per 30 µl sample (<10 pM), and aliquots were incubated with various concentrations of C1q in MgPBS for 10 min at 37°C. Samples were then filtered through nitrocellulose, the filters washed with buffer and dried under an infrared lamp, and counted with addition of scintillation fluid (Ecoscint A, National Diagnostics, Atlanta, GA). A background sample of RNA alone was run in parallel. To measure inhibition, RNA plus C1q plus inhibitor (e.g., the A-chain residue 14-26 site, SAP, β-amyloid peptide, CRP) were incubated for 10 min at 37°C, and then filtered. Filters were washed and counted.

Immune complexes (IC) were formed by mixing 620 µg BSA at equivalence with 1 ml of rabbit anti-BSA (Sigma, St. Louis, MO) plus PEG 8000 added to 1% final concentration, and then samples were incubated overnight at 4°C. The IC were pelleted by microfugation at 12,000 rpm for 10 min, washed five times with PBS, and suspended in 1 ml of MgPBS. For measurement of C1q RNA clone binding to Clq-immune complexes (Clq-IC), 20 µl of the purified [³²P]-RNA plus 20 µl of the IC were mixed with 20 µl of C1q at various concentrations at between 10⁻¹¹ and 10⁻⁷ M in MgPBS plus 1% Triton. Samples were incubated for 30 min at room temperature, microfuged, and the pellets and supernatants counted.

### F Hemolytic Assays

Complement consumption was measured by C4 hemolytic assay as described (46). All samples were diluted and the assay run in veronal-buffered saline containing calcium, magnesium and 1 % gelatin (GVB⁺⁺- complement buffer). For measurement of C4 consumption by β-amyloid peptide consumption, the peptide was added at 250 µg/ml to a 1/8 dilution of whole human serum and then incubated for 60 min at 37°C. The sample was then diluted for assay of C4 hemolytic activity. For assay of inhibition by C1q 2'-NH₂-RNA clones, the RNA was included in the initial β-amyloid peptide-whole human serum incubation mixture, and then C4 amounts assayed as above.

### EXAMPLE 2. RNA Ligands to C1q

### A. RNA SELEX

The pool of random 50n7-2'NH₂ RNA bound C1q by nitrocellulose filter assay with a K_{d} of 2.3 µM. For SELEX round 1, the C1q concentration was between 0.156-1.25 µM and the RNA concentration was 15 µM. Throughout the SELEX process, the RNA concentrations were maintained at approximately 10-fold greater than the concentration of C1q, which was reduced at each round with a final round 14 C1q concentration of 136 pM. Background binding of RNA to nitrocellulose filters remained low throughout the SELEX procedure, in part because RNA was pre-adsorbed with nitrocellulose filters. The binding of pool RNA to C1q improved at each round. The evolved round 14 pool RNA bound C1q with a K_{d}=670 pM, yielding an overall improvement in binding K_{d} of 3400-fold.

Bulk RNA was then cloned for sequence determination and evaluation of binding. Through comparison of binding at 0.1 and 0.5 nM C1q, individual clones were ranked, and clones with C1q binding above background were sequenced and are shown in Table 2 (SEQ ID NOS: 5-20). Family 1 contained 12 of the 19 total sequences. Family 2 contained three sequences. Both Family 3 and Family 4 contained two sequences. Both Family 1 and Family 2 sequences contain G-rich regions, and both have the repeated sequence motifs, GGAG and GGUG. The identity and homology of Family 1 members is greatest in the 5' half, which is G-rich. The C-rich 3' half has only short stretches of sequence homology, and these are shown only with inclusion of large gap regions. Sequences from all families can be folded to give stem-loop structures with extensive Watson-Crick base-pairing. Full binding curves for the highest affinity ligands yielded a K_{d} range from 290 pM to 3.9 nM; the high affinity ligands were found in all four sequence families. All of the binding curves were monophasic. The binding maximum is not 100% because of variable amounts of nucleic acid alterations taking place during purification. This is known because usually ligands can be bound to protein, extracted, and then re-bound, and give maximum binding approaching 100% (data not shown).

### B. Competition

Ligands from different families interact with the same or overlapping sites on C1q, as shown by cross-competition. This site is on the collagen-like region, at or near the A-chain 14-26 residue site, as shown by two lines of evidence. First, C1q when bound to IC still binds the ligand #50 (SEQ ID NO: 12); binding to immunoglobulin Fc would block the head region, but leave the collagen-like tail available, suggesting that SELEX ligands are bound to the tail. Second and more direct, ligand #50 is competed by proteins which are known to bind the A-chain residue 14-26 site, including SAP, β-amyloid peptide, and CRP. Finally, ligand #50 is competed by a peptide that has the same amino acid sequence as residues 14-26 on the A-chain. This result is further supported by results for hemolytic inhibition as described below.

### C. Consumption

SELEX ligand binding to the A-chain 14-26 amino acid site could activate C1q, or, alternatively, SELEX ligands could inhibit the binding of other molecules and prevent C1q activation. This was tested by measuring C4 consumption in serum after incubation with a SELEX ligand, or after incubation with a known C1q activator together with a SELEX ligand. SELEX ligands when incubated in serum do not consume C4, and thus are not C1q activators. Nor do these ligands at this concentration inhibit serum lysis of antibody-coated sheep erythrocytes, which would occur if ligands bound near the C1q head groups (data not shown). The ligands do inhibit C4 consumption by another C1q activator, the β-amyloid 1-40 peptide. This peptide is known to activate C1q through binding at the A-chain 14-26 residue site; therefore, this inhibition confirms that SELEX ligands binds at this A-chain site. Control ligands from the SELEX process that did not bind C1q by nitrocellulose assay, were also ineffective in blocking the β-amyloid 1-40 peptide C1q activation.

### EXAMPLE 3. Nucleic Acid Ligands of Complement System Protein C3.

In order to generate ligands to complement protein C3, a library of about 10¹⁴ RNA or DNA molecules is generated that contains 50 or 30 nucleotides of contiguous random sequence flanked by defined sequences. In one embodiment, the random nucleotides of the initial Candidate Mixture are comprised of 2'-NH₂ pyrimidine bases. The rounds of selection and amplification are carried out as described supra in Example 1 using art-known techniques.

### EXAMPLE 4. Nucleic Acid Ligands of Complement System Protein C5.

In order to generate ligands to complement protein C5, a library of about 10¹⁴ RNA or DNA molecules is generated that contains 50 or 30 nucleotides of contiguous random sequence flanked by defined sequences. In one embodiment, the random nucleotides of the initial Candidate Mixture are comprised of 2'-NH₂ pyrimidine bases. The rounds of selection and amplification are carried out as described supra in Example 1 using art-known techniques.

### EXAMPLE 5. Activation of the Complement System.

Oligonucleotides can activate both classical and alternative pathways. Generally, poly-G containing oligonucleotides that form G-quartets are efficient activators. These poly-G oligonucleotides can interact with the basic site on the C1q collagen-like region, and since the poly-G structures form high molecular weight aggregates, they bind and activate C1q. Phosphorothioate oligonucleotides, which have increased non-specific binding as compared with phosphodiester oligonucleotides, are also efficient Complement System activators. Poly-G containing phosphorothioates are highly effective and even G-poor sequences can activate. The site on C1q where poly-G binding occurs is likely the same as the Nucleic Acid binding. The results for oligonucleotide activation are shown below where classical pathway activation is measure by C4d fragment ELISA (Quidel, San Diego, CA), and alternative pathway is measure by Bb ELISA (Quidel, San Diego, CA). Although separate pathways, there is evidence to suggest that oligonucleotide activation of both pathways is C1q dependent.

| **Sample** | | **[4d]** µ**g** | **[Bb**] µg |
|---|---|---|---|
| | | **(Class.)** | **(Altern.)** |
| Poly-AG | Random Co-Polymer | 8.1 | 18.9 |
| Poly-G | Random Co-Polymer | 1.2 | 29.3 |
| Poly-I | Random Co-Polymer | 0 | 14.7 |
| Poly-A | Random Co-Polymer | 0 | 0 |
| Poly-U | Random Co-Polymer | 0 | 1.8 |
| Poly-C | Random Co-Polymer | 0 | 2.5 |
| Phosphorothioate Oligonucleotides | | | |
| GGC**GGG**GCTACGTACC**GGGG**CTTTTGTAAAACCCCGCC SEQ ID NO.21 | | -7.1 | 32.4 |
| CTCTCGCACCCATCTCTCTCCTTCT SEQ ID NO: 22 | | 0.0 | 3.9 |
| BSA-anti-BSA | Immune Complexes | 8.0 | 11.9 |
| β-amyloid Peptide | | 2.7 | n/d |
| Fucoidan | Sulfated Carbohydrate | | 27 |
| buffer | | 0.0 | 0.0 |

Activation is also tested by hemolytic assays. Known activators, including 2'-OH poly-G and Phosphorothioate oligonucleotides, as well as potential activators such as multimerized C1q Nucleic Acid Ligands and small (e.g., 15-mer) 2'-F poly-G oligonucleotides are coated on sheep erythrocytes and subsequent lysis of the erythrocytes by serum complement is measured. Methods of coating oligonucleotides and Nucleic Acid Ligands on cells include passive adsorption, chemical conjugation, streptavidin-biotin coupling, and specific Nucleic Acid binding. Following treatment with fresh rat or human serum, the deposition of complement components on the cell, membrane damage and lysis are measured by standard methods as would be known by one of skill in the art.

Complement activation by oligonucleotides may be related to oligonucleotide multimerization. Poly-G 2'-OH oligonucleotides form large aggregates, and if coated on cells activate serum complement and cause lysis. Poly-G on cells may form larger aggregates and activate more efficiently than in solution. In addition, phosphorothioate oligonucleotides that self-associate and bind non-specifically to cell surfaces trigger high levels of complement activation lysis. Smaller oligonucleotides such as 15-mer 2'-F poly-G may not activate in solution, but be induced to form activating aggregates on a cell. These properties are useful in designing semi-specific activating oligonucleotides. C1q Nucleic Acid Ligands that do not activate in solution activate on cells if multimerized prior to cell binding, or induced to multimerize on the cell; the latter provides the greatest specificity. The C1q Nucleic Acid Ligands when bound on cells may self-associate through their G-rich regions, forming triplexes and quartet structures, thereby becoming activators of the Complement System.

### A. Aggregation of C1q Nucleic Acid Ligands

C1q Nucleic Acid Ligands are dimerized using chemical cross-linkers of various lengths. Alternatively, Nucleic Acid Ligand monomers are biotinylated and then multimerized with streptavidin. Each of these multimers are tested for complement activation and lysis of erythrocytes.

Nucleic Acid Ligands are also synthesized in pairs with the addition of spacers and short complementary sequences in such a configuration that when mixed together, they form Nucleic Acid dimers that are capable of binding at two sites on C1q and activating the Complement System. Nucleic Acid Ligands from different families can be used to form heterodimers, or Nucleic Acid Ligands from the same family can be used to form homodimers.

An oligonucleotide-dendrimer is synthesized that contains multiple short hybridization sequences. Complementary sequences are then incorporated into the C1q Nucleic Acid Ligands in a manner that does not affect the binding of the Nucleic Acid Ligand to C1q. The combination of the dendrimer and the C1q Nucleic Acid Ligand on the cell surface forms multimers which trigger the Complement System. The dendrimers are added first to allow them to associate with the cell surface. C1q Nucleic Acid Ligands are then added and associate with the dendrimers on the cell surface. Adding the dendrimers and C1q Nucleic Acid Ligands separately prevents activation of the Complement System before reaching the targeted cell(s), and thus provides specificity.

The addition of poly-G sequence to C1q Nucleic Acid Ligands provides additional binding ability. In addition, short poly-G sequences on individual C1q Nucleic Acid Ligands forms higher order structures, which serve to multimerize the C1q Nucleic Acid Ligands and cause activation.

### B. Lysis of Erythrocytes and Leukocytes

Nucleic Acid Ligands that promote erythrocyte lysis are tested on nucleated cells, including lymphocytes and tumor cells. Nucleated cells have mechanisms of complement resistance that erythrocytes lack; for example, nucleated cells can shed antigens, bleb off membrane vesicles containing the complement components, and express increased levels of complement inhibitors as compared with erythrocytes and may up-regulate protective mechanisms upon initial complement attack. As high levels of activation are important for cell killing, activators are compared for amount of Complement System component deposition and extent of membrane damage. Also, different types and sources of tumor cells and lymphocytes are tested to determine if susceptibility is cell-type specific.

Nucleic Acid Ligands can be generated for virtually any target as described in the SELEX Patent Applications. Nucleic Acid Ligands to L-Selectin have been generated (See United States Patent Application Serial No. 08/479,724, filed June 7, 1995, entitled "High Affinity Nucleic Acid Ligands to Lectins,"). The diversity of lectin mediated functions provides a vast array of potential therapeutic targets for lectin antagonist. For example, antagonists to the mammalian selectins, a family of endogenous carbohydrate binding lectins, may have therapeutic applications in a variety of leukocyte-mediated disease states. Inhibition of selectin binding to its receptor blocks cellular adhesion and consequently may be useful in treating inflammation, coagulation, transplant rejection, tumor metastasis, rheumatoid arthritis, reperfusion injury, stroke, myocardial infarction, bums, psoriasis, multiple sclerosis, bacterial sepsis, hypovolaemic and traumatic shock, acute lung injury and ARDS. The coupling of C1q Nucleic Acid Ligands to L-Selectin Nucleic Acid Ligands makes the L-selectin Nucleic Acid Ligand more efficient by promoting cell killing at the target. C1q Nucleic Acid Ligands are coupled to L-Selectin Nucleic Acid Ligands, and the conjugates are tested for leukocyte lysis as described above. Also, Nucleic Acid Ligands to other cell surface targets, antibodies to all targets that do not themselves activate complement, cytokines, growth factors, or a ligand to a cell receptor could be coupled to a C1q Nucleic Acid Ligand and used for cell killing.

### C. The Mechanism(s) of Poly-G and Phosphorothioate Complement Activation

The mechanism(s) of poly-G and phosphorothioate complement activation are determined, for both classical and alternative pathways, by inhibition with C1q Nucleic Acid Ligands and by use of antibody and depleted-sera reagents.

Some activating oligonucleotides function through the alternative rather than the classical pathway, and the characteristics of these oligonucleotides are determined. The most important alternative pathway component is C3; deposition of C3 on a surface initiates the alternative pathway and degradation of C3 shuts it down. Oligonucleotides show increased binding to the activated, i.e., proteolysed, form of C3, and the oligonucleotides may therefore initiate activation. This is tested by specific assays with polynucleotides, and by making Nucleic Acid Ligands to C3 as described in Example 3.

### D. In Vivo Testing of Complement Activation

Nucleic Acid Ligand-mediated Complement System activation is tested in animals to evaluate in vivo Nucleic Acid Ligand action. Erythrocytes and/or lymphocytes are coated with Nucleic Acid Ligands and injected into rats to test cell killing and lysis in vivo. Activating Nucleic Acid Ligands are also coupled to a MoAb that does not activate the Complement System, where the antibody is directed against a rat cell antigen (e.g., lymphocyte antigen). These calls are then coated with the Nucleic Acid Ligand-antibody conjugate and injected into rats. Alternatively, the Nucleic Acid Ligand -antibody conjugate is injected directly into the rat and then in vivo leukocyte killing is measured.

It is also possible that C1q Nucleic Acid Ligands cross-react with non-human C1q, and non-human C1q could be used for in vivo assays. C1q Nucleic Acid Ligands are tested against species such as mouse, rat and rabbit C1q. C1q is purified from serum and cross-reactivity with C1q Nucleic Acid Ligands is tested by nitrocellulose binding assay. Alternatively, C1q is bound to immune complexes which are added to serum and then C1q Nucleic Acid Ligand binding to the aggregate is tested. If Nucleic Acid Ligands are species-specific, then rat serum is depleted of rat C1q by continuous perfusion over a Ig-Sepharose column, and the serum is reconstituted with human C1q by methods known to one of skill in the art. These reconstituted animals are then used to test C1q Nucleic Acid Ligands for targeted Complement System activation and cell killing.

### LITERATURE CITED

1. Cooper, N. R. 1985. The classical complement pathway: activation and regulation of the first complement component. Adv. Immunol. 37:151.
2. Reid, K. B. M. and S. Thiel. 1993. C1q and related molecules in defence. In The Natural Immune System: Humoral Factors. E. Sim, ed. IRL Press, Oxford, p. 151.
3. Jiang, H., J. N. Siegel, and H. Gewurz. 1991. Binding and complement activation by C-reactive protein via the collagen-like region of C1q and inhibition of these reactions by monoclonal antibodies to C-reactive protein and C1q. J. Immunol. 146:2324.
4. Bristow, C. L. and R. Boackle. 1986. Evidence for the binding of human serum amyloid P component to C1q and Fab. Mol. Immunol. 23:1045.
5. Schultz, J., J. Schaller, M. McKinley, B. Bradt, N. Cooper, P. May, and J. Rogers. 1994. Enhanced cytotoxicity of amyloid b-peptide by a complement dependent mechanism. Neurosci. Lett. 175:99.
6. Snyder, S. W., G. T. Wang, L. Barrett, U. S. Ladror, D. Casuto, C. M. Lee, G. A. Krafft, R. B. Holzman, and T. F. Holzman. 1994. Complement C1q does not bind monomeric b-amyloid. Exp. Neurol. 128:136.
7. Jiang, H., D. Burdick, C. G. Glabe, C. W. Cotman, and A. J. Tenner. 1994. b-amyloid activates complement by binding to a specific region of the collagen-like domain of the C1q A chain. J. Immunol. 152:5050.
8. Eikelenboom, P. and F. C. Stam. 1982. Immunoglobulins and complement factors in senile plaques: an immunoperoxidase study. Acta Neuropath. 57:239*.*
9. Eikelenboom, P., C. E. Hack, J. M. Rozemuller, and R. C. Starn. 1989. Complement activation in amyloid plaques in Alzheimer's dementia. Virchows Arch. [B] 56:259.
10. Rogers, J., N. R. Cooper, S. Webster, J. Schultz, P. L. McGeer, S. D. Styren, W. H. Civin, L. Brachova, B. Bradt, P. Ward, and I. Lieberburg. 1992. Complement activation by b-amyloid in Alzheimer disease. Proc. Natl. Acad. Sci. USA 89:10016.
11. Dietzschold, B., W. Schwaeble, M. K. H. Schäfer, D. C. Hooper, Y. M. Zehng, F. Petry, H. Sheng, T. Fink, M. Loos, H. Koprowski, and E. Weihe. 1995. Expression of C1q, a subcomponent of the rat complement system, is dramatically enhanced in brains of rats with either Borna disease or experimental allergic encephalomyelitis. J. Neurol. Sci. 130:11.
12. Reid, K. B. M. and J. Edmondson. 1984. Localization of the biding site in subcomponent C1q for plasma fibronectin. Acta Pathol. Microbiol. Immunol. Scand. Sect. C 92 (Suppl. 284):11.
13. Bohnsack, J. F., A. J. Tenner, G. W. Laurie, H. K. Kleinman, G. R. Martin, and E. J. Brown. 1985. The C1q subunit of the first component of complement binds to laminin: A mechanism of the deposition and retention of immune complexes in basement membrane. Proc. Natl. Acad. Sci. USA 82:3824.
14. Entwistle, R. A. and L. T. Furcht. 1988. C1q Component of Complement Binds to Fibrinogen and Fibrin. Biochem. 27:507.
15. Stoiber, H., C. F. Ebenbichler, N. M. Thielens, G. J. Arlaud, and M. P. Dierich. 1995. HIV-1 rsgp41 depends on calcium for binding of human C1q but not for binding of gp120. Mol. Immunol. 32:371.
16. Nishioka, M., K. Kobayashi, M. Uchida, and T. Nakamura. 1982. A binding activity of actin with Human C1q. Biochem. Biophys. Res. Commun. 108:1307.
17. Koethe, S. M., K. E. Nelson, and C. G. Becker. 1995. Activation of the classical pathway of complement by tobacco glycoprotein (TGP). J Immunol. 155:826.
18. Hughes-Jones, N. C. and B. Gardner. 1978. The reaction between the complement subcomponent C1q, IgG complexes and polyionic molecules. Immunology 34:459.
19. Almeda, S., R. D. Rosenberg, and D. H. Bing. 1983. The binding Properties of Human Complement Component C1q. Interaction with Mucopolysaccharides. J Biol. Chem. 258:785.
20. Blondin, C., E. Fischer, C. Boisson-Vidal, M. D. Kazatchkine, and J. Jozefonvicz. 1994. Inhibition of complement activation by natural sulfated polysaccharides (fucans) from brown seaweed. Mol. Immunol. 31:247.
21. Silvestri, L., J. R. Baker, L. Roden, and R. M. Stroud. 1981. The C1q inhibitor in Serum is a Chrondroitin 4-Sulfate Proteoglycan. J. Biol. Chem. 256:7383.
22. Zohair, A., S. Chesne, R. H. Wade, and M. G. Colomb. 1989. Interaction between complement subcomponent C1q and bacterial lipopolysaccharides. Biochem. J. 257:865.
23. Stoiber, H., N. M. Thielens, C. Ebenbichler, G. J. Arlaud, and M. P. Dierich. 1994. The envelope glycoprotein of HIV-1 gp120 and human complement protein C1q bind to the same peptides derived from three different regions of gp41, the transmembrane glycoprotein of HIV-1, and share antigenic homology. Eur. J. Immunol. 24:294.
24. Schravendijk, M. R. and R. A. Dwek. 1982. Interaction of C1q with DNA. Mol. Immunol. 19:1179.
25. Rosenberg, A. M., P. A. Prokopchuk, and J. S. Lee. 1988. The binding of native DNA to the collagen-like segment of C1q. J. Rheumatol. 15:1091.
26. Uwatoko, S. and M. Mannik. 1990. The location of binding sites on C1q for DNA. J Immunol. 144:3484.
27. Acton, S., D. Resnick, M. Freeman, Y. Ekkel, J. Ashkenas, and M. Krieger. 1993. The collagenous domains of macrophage scavenger receptors and complement component C1q mediate their similar, but not identical, binding specificities for polyanionic ligands. J. Biol. Chem. 268:3530.
28. Linder, E. 1981. Binding of C1q and complement activation by vascular endothelium. J Immunol. 126:648.
29. Pinckard, R. N., M. S. Olson, R. E. Kelley, D. H. DeHeer, J. D. Palmer, R. A. O'Rourke, and S. Goldfein. 1973.. J. Immunol. 110:1376.
30. Storrs, S. B., W. P. Kolb, R. N. Pinckard, and M. S. Olson. 1981. Characterization of the Binding of Purified Human C1q to Heart Mitochondrial Membranes. J. Biol. Chem. 256:10924.
31. Giclas, P. C., R. N. Pinckard, and M. S. Olson. 1979. In Vitro activation of complement by isolated human heart subcellular membranes. J. Immunol. 122:146.
32. Storrs, S. B., W. P. Kolb, and M. S. Olson. 1983. C1q binding and C1 activation by various isolated cellular membranes. J. Immunol. 131:416.
33. Linder, E., V. -P. Lehto, and S. Stenman. 1979. Activation of complement by cytoskeletal intermediate filaments. Nature 278:176.
34. Li, Y. -P., C. Mold, and T. W. Du Clos. 1994. Sublytic complement attack exposes C-reactive protein binding sites on cell membranes. J. Immunol. 152:2995.
35. Ying, S-C., A. T. Gewurz, H. Jiang, and H. Gewurz. 1993. Human serum amyloid P component oligomers bind and activate the classical complement pathway via residues 14-26 and 76-92 of the A chain collagen-like region of C1q. J. Immunol. 150:169.
36. Newman, A. 1994. RNA splicing: Activity in the spliceosome. Curr. Biol. 4:462.
37. Jiang, H., B. Cooper, F. A. Robey, and H. Gewurz. 1992. DNA binds and activates complement via residues 14-26 of the human C1q A chain. J. Biol. Chem. 267:25597.
38. Yabkowitz, R., J. B. Lowe, and V. M. Dixit. 1989.. J. Biol. Chem. 264:10888.
39. Cardin, A. D. and H. J. R. Weintraub. 1989.. Arteriosclerosis 9:21*.*
40. Deprez, P. N. and N. C. Inestrosa. 1995. Two heparin-binding domains are present on the collagenic tail of asymmetric acetylcholinesterase. J Biol. Chem 270:11043.
41. Tuerk, C. and L. Gold. 1990. Systematic Evolution of Ligands by Exponential Enrichment: RNA Ligands to Bacteriophage T4 DNA Polymerase. Science 249:505.
42. Jellinek, D., L. S. Green, C. Bell, C. K. Lynott, N. Gill, C. Vargeese, G. Kirschenheuter, D. P. C. McGee, P. Abesinghe, W. A. Pieken, R. Shapiro, D. B. Rifkin, D. Moscatelli, and N. Janjic. 1995. Potent 2'-amino-2'-deoxypyrimidine RNA inhibitors of basic fibroblast growth factor. Biochem. 34:11363.
43. Jellinek, D., L. S. Green, C. Bell, and N. Janjic. 1994. Inhibition of receptor binding by high-affinity RNA ligands to vascular endothelial growth factor. Biochem. 33:10450.
44. Milligan, J. F., D. R. Groebe, G. W. Witherell, and O. Uhlenbeck. 1987.. Nucleic Acids Res. 12:785.
45. Sambrook, J., E. F. Fritsch, and T. Maniatis. 1989. Molecular Cloning. A laboratory Manual. Cold Spring Harbor Laboratory, Cold Spring Harbor, NY.
46. Gaither, T. A., D. W. Alling, and M. M. Frank. 1974. A new one-step method for the functional assay of the fourth component (C4) of human and guinea pig complement. J. Immunol. 113:574.
47. Janeway, Charles A., Jr. and P. Travers. 1994. Immunobiology: The Immune System in Health and Disease. Current Biology Ltd, San Francisco, pp. 8:35-8:55.
48. Morgan, B. P. (1995) Physiology and pathophysiology of complement: progress and trends. Crit. Rev. in Clin Lab. Sci. 32(3):265-298.
49. Menzel, E. -J., J. Smolen, and K. Reid (1981) Interaction of collagen with C1q via its collagen-like portion. Biochim. Biophys. Acta 670:265.
50. Matis, L. A., and S. A. Rollins (1995) Complement-specific antibodies: designing novel anti-inflammatories. Nature Medicine 1(8):839-842.

**TABLE 1**

| SEQ ID NO. | For RNA SELEX: |
|---|---|
| | Synthetic DNA Template: |
| 1 | 5'-TAATACGACTCACTATAGGGAGGACGATGCGG- [N]50 -CAGACGACTCGCCCGA-3' |
| | Starting random sequence RNA pool: |
| 2 | 5'-GGGAGGACGAUGCGG- [N]50 -CAGACGACUCGCCCGA-3' |
| | Primer Set: |
| 3 | 5'-PRIMER: 5'-TAATACGACTCACTATAGGGAGGACGATGCGG-3' |
| 4 | 3'-PRIMER: 5'-TCGGGCGAGTCGTCTG-3' |

**TABLE 2**

| | | | 2'-NH₂ RNA Ligands of Complement System Protein Clq* |
|---|---|---|---|
| SEQ ID NO. | Kd(nM) | Clone No. | Family 1 |
| 5 | | 3 | gggaggacgaugcggGAGGAGUGGAGGUAAACAAUAGGUCGGUAGCGACUCCCACUAACAGGCCUcagacgacucgcccga |
| 6 | | 12 | gggaggacgaugcggGUGGAGUGGAGGUAAACAAUAGGUCGGUAGCGACUCCCAGUAACGGCCUcagacgacucgcccga |
| 7 | | 23c | gggaggacgaugcaaGUGGAGUGGAGGUAUAACGGCCGGUAGGCAUCCCACUCGGGCCUAGCUcagacgacucgcccga |
| 8 | | 30 | gggaggacgaugcggGUGGAGUGGGGAUCAUACGGCUGGUAGCACGAGCUCCCUAACAGCGGUcagacgacucgcccga |
| 9 | 0.29 | 36 | gggaggacgaugcggGAGGAGUGGAGGUAAACAAUAGGCCGGUAGCGACUCCCACUAACAGCCUcagacgacucgcccga |
| 10 | 1.38 | 45 | gggaggacgaugcggUGGAGUGGAGGUAUACCGGCCGGUAGCGCAUCCCACUCGGGUCUGUGCUcagacgacucgcccga |
| 11 | | 47 | gggaggacgaugcggGUGGAGCGGAGGUUUAUACGGCUGGUAGCUCGAGCUCCCUAACACGCGGUagacgacucgcccga |
| 12 | 0.979 | 50 | gggaggacgaugcggGUGGAGUGGAGGUAUAACGGCCGGUAGCGCAUCCCACUCGGGUCUGUGCUagacgacucgcccga |
| 13 | | 78 | gggaggacgaugcggGUGGAGUGGAGGGUAAACAAUGGCUGGUGGCAUUCGGAAUCUCCCAACGUagacgacucgcccga |
| | | | Family 2 |
| 14 | 3.85 | 33 | gggaggacgaugcggGUUGCUGGUAGCCUGAUGUGGGUGGAGUGAGUGGAGGGUUGAAAAAUGcagacgacucgcccga |
| 15 | | 40 | gggaggacgaugcggCUGGUAGCAUGUGCAUUGAUGGGAGGAGUGGAGGUCACCGUCAACCGUcagacgacucgcccga |
| 16 | | 43 | gggaggacgaugcggUUUCUCGGCCAGUAGUUUGCGGGUGGAGUGGAGGUAUAUCUGCGUCCUCGcagacgacucgcccga |
| | | | Family 3 |
| 17 | 2.4 | 14 | gggaggacgaugcggCACCUCACCUCCAUAUUGCCGGUUAUCGCGUAGGGUGAGCCCAGACACGAcagacgacucgcccga |
| 18 | | 23 | gggaggacgaugcggCACUCACCUUCAUAUUGGCCGCCAUCCCCAGGGUUGAGCCCAGACACAGcagacgacucgcccga |
| | | | Family 4 |
| 19 | | 22 | gggaggacgaugcggGCAUAGUGGGCAUCCCAGGGUUGCCUAACGGCAUCCGGGGUUGUUAUUGGcagacgacucgcccga |
| 20 | | 67 | gggaggacgaugcggCAGACGACUCGCCCGAGGGGAUCCCCCGGGCCUGCAGGAAUUCGAUAUcagacgacucgcccga |

| | | | |
|---|---|---|---|
| * Lower case letters represent the fixed region. | | | |

## Claims

1. A non-naturally occurring nucleic acid ligand that binds specifically and with high affinity to Complement System Protein C1q and which is an inhibitor of activation of the complement system, wherein the nucleic acid ligand is
(a) SEQ ID NO: 12 or
(b) a nucleic acid ligand that has substantially the same ability to bind C1q and inhibit C1q mediated activation of the complement system as SEQ ID NO: 12, wherein the nucleic acid ligand shares a degree of primary sequence homology in excess of 70% with a nucleic acid ligand selected from the group consisting of SEQ ID NOs: 5-20.

2. A ligand according to claim 1 wherein the nucleic acid ligand is RNA.

3. A ligand according to claim 1 or 2 wherein the nucleic acid ligand is chemically modified.

4. A ligand according to claim 3 wherein the chemical modification is chosen from:
(i) 2'-position sugar modifications;
(ii) 5-position pyrimidine modifications;
(iii) 8-position purine modifications;
(iv) modification at exocyclic amines;
(v) substitution of 4-thiouridine;
(vi) substitution of 5-bromo-uracil;
(vii) substitution of 5-iodo-uracil;
(viii) backbone modifications;
(ix) posphorothioate modifications;
(x) alkyl phosphate modifications;
(xi) methylations;
(xii) 3' capping;
(xiii) 5' capping.

5. A ligand according to claim 1 or 2 wherein the nucleic acid ligand is 2'-NH₂ RNA.

6. A pharmaceutical comprising a ligand according to any one of claims 1 to 5.

7. A ligand or pharmaceutical according to any one of claims 1 to 6 for use in the treatment or prevention of disease, wherein said disease is caused by activation of the complement system.

8. Use of a ligand according to any one of claims 1 to 5 in the manufacture of a pharmaceutical for treatment or prevention of disease, wherein said disease is caused by activation of the complement system.

9. A ligand according to any one of claims 1 to 5 for use as an in vitro diagnostic agent.

10. A ligand according to any one of claims 1 to 5 for use as an in vivo diagnostic agent.

11. Use of a ligand according to any one of claims 1 to 5 in the manufacture of a diagnostic agent for diagnosis of disease.

## Patentansprüche

1. Nicht natürlich vorkommender Nucleinsäureligand, der spezifisch und mit hoher Affinität an das Protein C1 q des Komplementsystems bindet und ein Inhibitor der Aktivierung des Komplementsystems ist, worin der Nucleinsäureligand
(a) Seq.-ID Nr. 12 aufweist oder
(b) ein Nucleinsäureligand ist, der im Wesentlichen die gleiche Fähigkeit zur Bindung an C1q und zur Hemmung von C1q-vermittelter Aktivierung des Komplementsystems aufweist wie Seq.-ID Nr. 12, worin der Nucleinsäureligand mehr als 70 % Primärsequenzhomologie mit einem Nucleinsäureliganden aufweist, der aus der aus Seq.-ID Nr. 5-20 bestehenden Gruppe ausgewählt ist.

2. Ligand nach Anspruch 1, worin der Nucleinsäureligand RNA ist.

3. Ligand nach Anspruch 1 oder 2, worin der Nucleinsäureligand chemisch modifiziert ist.

4. Ligand nach Anspruch 3, worin die chemische Modifikation ausgewählt ist aus:
(i) Zuckermodifikationen an der 2' -Position;
(ii) Pyrimidinmodifikationen an der 5-Position;
(iii) Purinmodifikationen an der 8-Position;
(iv) einer Modifikation an exozyklischen Aminen;
(v) einer Substitution von 4-Thiouridin;
(vi) einer Substitution von 5-Bromuracil;
(vii) einer Substitution von 5-loduracil;
(viii) Rückgratmodifikationen;
(ix) Thiophosphatmodifikationen;
(x) Alkylphosphatmodifikationen;
(xi) Methylierungen;
(xii) 3' -Capping;
(xiii) 5' -Capping.

5. Ligand nach Anspruch 1 oder 2, worin der Nucleinsäureligand 2' -NH₂-RNA ist.

6. Pharmazeutikum, das einen Liganden nach einem der Ansprüche 1 bis 5 umfasst.

7. Ligand oder Pharmazeutikum nach einem der Ansprüche 1 bis 6 zur Verwendung bei der Behandlung oder Prävention einer Erkrankung, worin die Erkrankung durch Aktivierung des Komplementsystems ausgelöst wird.

8. Verwendung eines Liganden nach einem der Ansprüche 1 bis 5 zur Herstellung eines Pharmazeutikums zur Behandlung oder Prävention einer Erkrankung, worin die Erkrankung durch Aktivierung des Komplementsystems ausgelöst wird.

9. Ligand nach einem der Ansprüche 1 bis 5 zur Verwendung als In-vitro-Diagnostikum.

10. Ligand nach einem der Ansprüche 1 bis 5 zur Verwendung als In-vivo-Diagnostikum.

11. Verwendung eines Liganden nach einem der Ansprüche 1 bis 5 zur Herstellung eines Diagnostikums zur Diagnose einer Erkrankung.

## Revendications

1. Ligand d'acide nucléique se produisant non-naturellement qui se lie spécifiquement et avec une affinité élevée à la Protéine C1q du Système du Complément et qui est un inhibiteur d'activation du système du complément, où le ligand d'acide nucléique est
(a) SEQ ID NO: 12 ou
(b) un ligand d'acide nucléique qui a sensiblement la même aptitude à se lier à C1q et à inhiber l'activation régulée par C1q du système du complément comme SEQ ID NO: 12, où le ligand d'acide nucléique partage un degré d'homologie de séquence primaire dépassant 70% avec un ligand d'acide nucléique sélectionné dans le groupe consistant en SEQ ID Nos: 5-20.

2. Ligand selon la revendication 1, où le ligand d'acide nucléique est ARN.

3. Ligand selon la revendication 1 ou 2, où le ligand d'acide nucléique est chimiquement modifié.

4. Ligand selon la revendication 3, où la modification chimique est sélectionnée parmi:
(i) modifications du sucre en position 2';
(ii) modifications de la pyrimidine en position 5;
(iii) modifications de la purine en position 8;
(iv) modification aux amines exocycliques;
(v) substitution de 4-thiouridine;
(vi) substitution de 5-bromo-uracile;
(vii) substitution de 5-iodo-uracile;
(viii) modifications de l'ossature;
(ix) modifications de phosphorothioate;
(x) modifications d'alkyl phosphate;
(xi) méthylations;
(xii) capping en 3';
(xiii) capping en 5'.

5. Ligand selon la revendication 1 ou 2, où le ligand d'acide nucléique est 2'-NH₂ ARN.

6. Médicament comprenant un ligand selon l'une quelconque des revendications 1 à 5.

7. Ligand ou médicament selon l'une quelconque des revendications 1 à 6 pour utilisation dans le traitement ou la prévention d'une maladie, où ladite maladie est provoquée par l'activation du système du complément.

8. Utilisation d'un ligand selon l'une quelconque des revendications 1 à 5 dans la fabrication d'un médicament pour le traitement ou le prévention d'une maladie, où ladite maladie est provoquée par l'activation du système du complément.

9. Ligand selon l'une quelconque des revendications 1 à 5 pour utilisation comme agent de diagnostic in vitro.

10. Ligand selon l'une quelconque des revendications 1 à 5 pour utilisation comme agent de diagnostic in vivo.

11. Utilisation d'un ligand selon l'une quelconque des revendications 1 à 5 dans la fabrication d'un agent de diagnostic pour le diagnostic d'une maladie.


### SEQUENCE LISTING

(1) GENERAL INFORMATION:
   (i) APPLICANT:
      NEXSTAR PHARMACEUTICALS, INC.
      BIESECKER, GREGORY
      GOLD, LARRY
   (ii) TITLE OF INVENTION: HIGH AFFINITY NUCLEIC ACID LIGANDS OF COMPLEMENT SYSTEM PROTEINS
   (iii) NUMBER OF SEQUENCES: 22
   (iv) CORRESPONDENCE ADDRESS:
      (A) ADDRESSEE: Swanson & Bratschun, L.L.C.
      (B) STREET: 8400 E. Prentice Place #200
      (C) CITY: Denver
      (D) STATE: Colorado
      (E) COUNTRY: US
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/595,335
      (B) FILING DATE: 1 FEB 1996
      (C) CLASSIFICATION:
   (viii) ATTORNEY/AGENT INFORMATION:
      (A) NAME: Barry J. Swanson, Esq.
      (B) REGISTRATION NUMBER: 33,215
      (C) REFERENCE/DOCKET NO.: NEX50/PCT
   (ix) TELECOMMUNICATION INFORMATION:
      (A) TELEPHONE: (303) 793-3333
      (B) TELEFAX: (303) 793-3433
(2) INFORMATION FOR SEQ ID NO: 1:
   (i) SEQUENCE CHARACTERISTICS:
      (A) LENGTH: 98 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 1:
   TAATACGACT CACTATAGGG AGGACGATGC GGNNNNNNNN NNNNNNNNNN NNNNNNNNNN 60
   NNNNNNNNNN NNNNNNNNNN NNCAGACGAC TCGCCCGA 98
(2) INFORMATION FOR SEQ ID NO: 2:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA

   (xi) SEQUENCE DESCRIPTION: SEQ ID NO: 2:
(2) INFORMATION FOR SEQ ID NO: 3:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 3:
      TAATACGACT CACTATAGGG AGGACGATGC GG 32
(2) INFORMATION FOR SEQ ID NO: 4:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA

   (xi) SEQUENCE DESCRIPTION: SEQ ID NO: 4:
   TCGGGCGAGT CGTCTG 16
(2) INFORMATION FOR SEQ ID NO: 5:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 5:
   GGGAGGACGA UGCGGGAGGA GUGGAGGUAA ACAAUAGGUC GGUAGCGACU CCCACUAACA    60
   CGCCUCAGAC GACUCGCCCG A                                              81
(2) INFORMATION FOR SEQ ID NO: 6:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 6:
   GGGAGGACGA UGCGGGUGGA GUGGAGGUAA ACAAUAGGUC GGUAGCGACU CCCAGUAACG    60
   GCCUCAGACG ACUCGCCCGA                                                80
(2) INFORMATION FOR SEQ ID NO: 7:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 7:
   GGGAGGACGA UGCGGGUGGA GUGGAGGUAU AACGGCCGGU AGGCAUCCCA CUCGGGCCUA    60
   GCUCAGACGA CUCGCCCGA                                                 79
(2) INFORMATION FOR SEQ ID NO: 8:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 8:
   GGGAGGACGA UGCGGGUGGA GUGGGGAUCA UACGGCUGGU AGCACGAGCU CCCUAACAGC   60
   GGUCAGACGA CUCGCCCGA                                                79
(2) INFORMATION FOR SEQ ID NO: 9:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 9:
   GGGAGGACGA UGCGGGAGGA GUGGAGGUAA ACAAUAGGCC GGUAGCGACU CCCACUAACA   60
   GCCUCAGACG ACUCGCCCGA                                               80
(2) INFORMATION FOR SEQ ID NO: 10:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 10:
   GGGAGGACGA UGCGGUGGAG UGGAGGUAUA CCGGCCGGUA GCGCAUCCCA CUCGGGUCUG   60
   UGCUCAGACG ACUCGCCCGA                                               80
(2) INFORMATION FOR SEQ ID NO: 11:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 11:
   GGGAGGACGA UGCGGGUGGA GCGGAGGUUU AUACGGCUGG UAGCUCGAGC UCCCUAACAC   60
   GCGGUCAGAC GACUCGCCCG A                                             81
(2) INFORMATION FOR SEQ ID NO: 12:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 12:
   GGGAGGACGA UGCGGGUGGA GUGGAGGUAU AACGGCCGGU AGCGCAUCCC ACUCGGGUCU   60
   GUGCUCAGAC GACUCGCCCG A                                             81
(2) INFORMATION FOR SEQ ID NO: 13:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 13:
   GGGAGGACGA UGCGGGUGGA GUGGAGGGUA AACAAUGGCU GGUGGCAUUC GGAAUCUCCC   60
   AACGUCAGAC GACUCGCCCG A                                             81
(2) INFORMATION FOR SEQ ID NO: 14:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 14:
   GGGAGGACGA UGCGGGUUGC UGGUAGCCUG AUGUGGGUGG AGUGAGUGGA GGGUUGAAAA   60
   AUGCAGACGA CUCGCCCGA                                                79
(2) INFORMATION FOR SEQ ID NO: 15:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 15:
   GGGAGGACGA UGCGGCUGGU AGCAUGUGCA UUGAUGGGAG GAGUGGAGGU CACCGUCAAC   60
   CGUCAGACGA CUCGCCCGA                                                79
(2) INFORMATION FOR SEQ ID NO: 16:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 16:
   GGGAGGACGA UGCGGUUUCU CGGCCAGUAG UUUGCGGGUG GAGUGGAGGU AUAUCUGCGU 60
   CCUCGCAGAC GACUCGCCCG A 81
(2) INFORMATION FOR SEQ ID NO: 17:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 17:
   GGGAGGACGA UGCGGCACCU CACCUCCAUA UUGCCGGUUA UCGCGUAGGG UGAGCCCAGA 60
   CACGACAGAC GACUCGCCCG A 81
(2) INFORMATION FOR SEQ ID NO: 18:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 18:
   GGGAGGACGA UGCGGCACUC ACCUUCAUAU UGGCCGCCAU CCCCAGGGUU GAGCCCAGAC 60
   ACAGCAGACG ACUCGCCCGA 80
(2) INFORMATION FOR SEQ ID NO: 19:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: RNA
   (ix) FEATURE:
      (D) OTHER INFORMATION: All pyrimidines are 2'-NH₂ modified
   (xi) SEQUENCE DESCRIPTION: SEQ ID NO: 19:
   GGGAGGACGA UGCGGGCAUA GUGGGCAUCC CAGGGUUGCC UAACGGCAUC CGGGGUUGUU 60
   AUUGGCAGAC GACUCGCCCG A 81
(2) INFORMATION FOR SEQ ID NO: 20:
   (i) SEQUENCE CHARACTERISTICS:
      (A) LENGTH: 77 base pairs
      (B) TYPE: nucleic acid
      (C) STRANDEDNESS: single
      (D) TOPOLOGY: linear
   (ii) MOLECULE TYPE:RNA
   (ix) FEATURE:
      (D) OTHER INFORMATION: All pyrimidines are 2'-NH₂ modified
   (xi) SEQUENCE DESCRIPTION: SEQ ID NO: 20:
   GGGAGGACGA UGCGGCAGAC GACUCGCCCG AGGGAUCCCC GGGCCUGCAG GAAUUCGAUA 60
   UCAGACGACU CGCCCGA 77
(2) INFORMATION FOR SEQ ID NO: 21:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE:DNA
   (ix) FEATURE:
      (D) OTHER INFORMATION: All nucleotides are bound by a phosphorothioate linkage
   (xi) SEQUENCE DESCRIPTION: SEQ ID NO: 21:
      GGCGGGGCTA CGTACCGGGG CTTTGTAAAA CCCCGCC 37
(2) INFORMATION FOR SEQ ID NO: 22:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE:DNA
   (ix) FEATURE:
      (D) OTHER INFORMATION: All nucleotides are bound by a phosphorothioate linkage
   (xi) SEQUENCE DESCRIPTION: SEQ ID NO: 22:
      CTCTCGCACC CATCTCTCTC CTTCT 25